# EUROPEAN PATENT APPLICATION

(11) **EP 3 693 007 A1**
(43) Date of publication of application: **12.08.2020**
(21) Application number: 19206465.7
(22) Date of filing: 25.02.2014
(51) Int. Cl.: A61K 39/00, C07K 16/24, A61K 39/395, A61P 1/04

(54) **METHODS FOR TREATING CROHN'S DISEASE USING AN ANTI-IL23 ANTIBODY**

(30) Priority: 15.03.2013 US 201361789976 P
(62) Divisional of application: 14708465.1
(71) Applicant: Amgen, Inc, Thousand Oaks, CA 91320-1799 (US)
(72) Inventor: PAN, Wei-jian, Pullman, WA 99163-5263 (US); TSUJI, Wayne, Seattle, WA 98122 (US)
(74) Representative: Dörries, Hans Ulrich

(57) **Abstract**

The invention relates to products and methods for treating Crohn's disease. The products relate to antibodies that inhibit native human IL-23 while sparing IL-12. One example describes a Phase 1, randomized, double-blind, placebo-controlled, ascending multiple dose study to evaluate the safety, tolerability, pharmacokinetics and pharmacodynamics of an anti-IL-23 antibody (AMG 139) in healthy subjects and subjects with mild to severe Crohn's disease.

SEQUENCE LISTING

<110>  AMGEN INC.
       Pan, et al.

<120>  METHODS FOR TREATING CROHN'S DISEASE USING AN ANTI-IL23 ANTIBODY

<130>  32053/47591 PC

<150>  US-61/789,976
<151>  2013-03-15

<160>  2

<170>  PatentIn version 3.5

<210>  1
<211>  124
<212>  PRT
<213>  Homo sapiens

<400>  1

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Asn Glu Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Tyr Thr Ser Ser Trp Tyr Pro Asp Ala Phe Asp
            100                 105                 110

Ile Trp Gly Gln Gly Thr Met Val Thr Val Ser Ser
        115                 120

<210>  2
<211>  111
<212>  PRT
<213>  Homo sapiens

<400>  2

Gln Ser Val Leu Thr Gln Pro Pro Ser Val Ser Gly Ala Pro Gly Gln

1               5                   10                  15

Arg Val Thr Ile Ser Cys Thr Gly Ser Ser Ser Asn Thr Gly Ala Gly
            20                  25                  30

Tyr Asp Val His Trp Tyr Gln Gln Val Pro Gly Thr Ala Pro Lys Leu
        35                  40                  45

Leu Ile Tyr Gly Ser Gly Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Ser
                85                  90                  95

Leu Ser Gly Trp Val Phe Gly Gly Gly Thr Arg Leu Thr Val Leu
            100                 105                 110

## Description

### Field of the Invention

The invention relates to products and methods for treating Crohn's disease. The products relate to antibodies that inhibit native human IL-23 while sparing IL-12.

### Background

Crohn's disease (CD) is a nonspecific chronic transmural inflammatory disease that most commonly affects the distal ileum and colon, and may occur in any part of the gastrointestinal tract. Crohn's disease occurs most commonly between the ages of 15 and 35, although persons of any age may be affected. Patients with Crohn's disease have uncontrolled inflammation that causes direct or collateral damage to the intestinal mucosa. This inflammation can result either from persistence of inflammatory stimulus, due to impaired gut barrier function, or from a dysregulated inflammatory response (Sandborn, et al. Gastroenterology, 2008;135:1130-1141; Rutgeerts, P. Scand J Gastroenterol Suppl. 2003(237):30-33; Rutgeerts et al.,Aliment Pharmacol Ther. 2003;17(12):1435-1450).

IL-23 is a heterodimeric cytokine and a potent inducer of pro-inflammatory cytokines. IL-23 is related to the heterodimeric cytokine Interleukin 12 (IL-12) both sharing a common p40 subunit. In IL-23, a unique p19 subunit is covalently bound to the p40 subunit. In IL-12, the unique subunit is p35 (Oppmann et al., Immunity, 2000, 13: 713-715). IL-23 is expressed by antigen presenting cells (such as dendritic cells and macrophages) in response to activation stimuli such as CD40 ligation, Toll-like receptor agonists and pathogens. IL-23 binds a heterodimeric receptor comprising an IL-12Rβ1 subunit (which is shared with the IL-12 receptor) and a unique receptor subunit, IL-23R.

Studies in patients with Crohn's disease and ulcerative colitis have demonstrated that IL-23 is upregulated in diseased tissue, while IL-12 is not (Schmidt et al. Inflamm Bowel Dis. 2005;11(1):16-23). Genome-wide association studies in Crohn's disease and psoriasis patients showed significant association between the unique IL-23 receptor component, IL-23R, and disease (Cargill et al. Am J of Human Gen. 2007;80(2):273-290; Duerr et al. Science. 2006;314(5804):1461-1463). Furthermore, allelic variants of IL-23R have shown significant correlation with the frequency of ulcerative colitis (Cargill et al. Am J of Human Gen. 2007;80(2):273-290), rheumatoid arthritis (Farago et al. Ann of the Rheum Dis. 2008;67(2):248-250), ankylosing spondylitis (Burton et al. Nature Gen. 2007;39(11):1329-1337), and multiple sclerosis (Illes et al., Neuro Letters. 2008;431(1):36-38).

IL-23 acts on activated and memory T cells and promotes survival and expansion of the T cell subset, Th17. Th17 cells produce proinflammatory cytokines including IL-6, IL-17, TNFα, IL-22 and GM-CSF. IL-23 also acts on natural killer cells, dendritic cells and macrophages to induce pro-inflammatory cytokine expression. Unlike IL-23, IL-12 induces the differentiation of naive CD4+ T cells into mature Th1 IFNγ-producing effector cells, and induces NK and cytotoxic T cell function by stimulating IFNγ production. Th1 cells driven by IL-12 were previously thought to be the pathogenic T cell subset in many autoimmune diseases, however, more recent animal studies in models of inflammatory bowel disease, psoriasis, inflammatory arthritis and multiple sclerosis, in which the individual contributions of IL-12 versus IL-23 were evaluated have firmly established that IL-23, not IL-12, is the key driver in autoimmune/inflammatory disease (Ahern et al., Immun. Rev. 2008 226:147-159; Cua et al., Nature 2003 421:744-748; Yago et al., Arthritis Res and Ther. 2007 9(5): R96). It is believed that IL-12 plays a critical role in the development of protective innate and adaptive immune responses to many intracellular pathogens and viruses and in tumor immune surveillance. See Kastelein, et al., Annual Review of Immunology, 2007, 25: 221-42; Liu, et al., Rheumatology, 2007, 46(8): 1266-73; Bowman et al., Current Opinion in Infectious Diseases, 2006 19:245-52; Fieschi and Casanova, Eur. J. Immunol. 2003 33:1461-4; Meeran et al., Mol. Cancer Ther. 2006 5: 825-32; Langowski et al., Nature 2006 442: 461-5. As such, IL-23 specific inhibition (sparing IL-12 or the shared p40 subunit) is expected to have a potentially superior safety profile compared to dual inhibition of IL-12 and IL-23.

In preclinical models of inflammatory bowel disease (Ahern et al., Immun Rev. 2008;226:147-159), inflammatory arthritis (Yago et al. Arthritis Res and Ther. 2007;9(5):R96), and multiple sclerosis (MS) (Cua et al. Nature. 2003;421(6924):744-748), the beneficial effects of anti-IL-12/23p40 antibodies have been recapitulated through the blockade of IL-23 alone while sparing IL-12. In the clinic, anti-IL-12/23p40 antibodies (eg, ustekinumab and briakinumab) have been shown to induce clinical responses in CD (phase 2 study; Mannon et al. N Eng J of Med. 2004;351(20):2069-2079). These clinical data, together with human expression and genetic data, and data in mouse disease models, suggest that the therapeutic effects of the IL-12/23p40 antibodies may be due to the antagonism of IL-23, instead of IL-12.

Interleukin-12 is a major Th1-inducing cytokine and studies using IL-12/23p40 and IL-12p35 null mice suggest a dominant role for IL-12 in host defense against intracellular pathogens and in tumor immune surveillance (Bowman et al., Curr Op in Infect Dis. 2006;19(3):245-252; Langowski et al. Nature. 2006;442(7101):461-465; Meeran et al., Mol Cancer Ther. 2006;5(4):825-832; Fieschi and Casanova, Eur J of Immun. 2003;33(6):1461-1464).

Humans with autosomal recessive deficiency of either IL-12/23p40 or IL-12Rβ1 can neither produce nor respond to IL-12 or IL-23, have clinical disease caused by weakly virulent Mycobacteria (ie, bacillus Calmette-Guerin substrains and environmental species) and nontyphoid Salmonella species, and have severe forms of tuberculosis (Fieschi and Casanova, Eur J of Immun. 2003;33(6):1461-1464). Patients with deficiencies in IFNγ signaling (IFNGγR1, IFNγR2, or STAT1 nulls) have an overlapping susceptibility to these pathogens, suggesting that the IL-12/IFNγ axis is important for infectious immunity (Fieschi and Casanova, Eur J of Immun. 2003;33(6):1461-1464). Mice lacking only IL-23p19 have resistance to Mycobacteria tuberculosis and Salmonella enteritidis infection comparable to that of wild-type mice (Bowman et al., Curr Op in Infect Dis. 2006;19(3):245-252; Schulz et al. J Immunol. 2008;181(11):7891-7901). In contrast, IL-12/23p40 null and IL-12p35 null mice are much more susceptible to M tuberculosis and S enteritidis infection (Bowman et al., Curr Op in Infect Dis. 2006;19(3):245-252). It is possible that targeting either IL-12 or IL-23 may compromise immunity to certain pathogens. However, based on the dominance of the IL-12/IFNγ axis in mediating immunity to these pathogens, it is likely that neutralizing IL-23 while keeping IL-12 signaling intact may minimize the risk of infection.

There is growing evidence suggesting that IL-12-induced production of IFNγ is primarily involved in tumor suppression. Interleukin-12 promotes tumor immune surveillance and antitumor responses by inducing development of Th1 cells and proliferation and cytotoxic activity of CD8⁺ T cells and NK cells. Moreover, IL-12 regulates DNA repair mechanisms in UV radiation-induced skin tumors and malignant transformation of papillomas to carcinomas (Meeran et al., Mol Cancer Ther. 2006;5(4):825-832). In tumor models comparing IL-12p35-, IL-12/23p40- and IL-23p19-deficient mice, it was shown that IL-23p19 null mice were resistant to tumor formation, while IL-12p35 null and/or IL-12/23p40 null mice had increased tumor formation compared to wild type control mice (Langowski et al. Nature. 2006;442(7101):461-465). Treatment with an anti-IL-23-specific neutralizing antibody resulted in a decreased risk of tumor formation and faster elimination of injected tumor cells. In contrast, animals treated with an anti-IL-12/23p40 antibody had a significantly increased tumor incidence and burden (Langowski et al. I Nature. 2006;442(7101):461-465). Taken together, IL-23 appears to play a role in tumor formation through a decrease in CD8⁺ T cell activity and an increase in inflammatory infiltration and angiogenesis in the tumor microenvironment, while IL-12 appears to be important for tumor suppression.

Targeting Crohn's disease is supported by robust genetic and nonclinical data, and by the demonstrated clinical efficacy of anti-IL-12/23p40 antibodies (ustekinumab and briakinumab) in Crohn's disease (Sandborn, et al. Gastroenterology, 2008; 135:1130-1141; Mannon et al. N Eng J of Med. 2004; 351(20):2069-2079). Mice deficient in IL-23p19-synthesis are protected against experimental colitis while mice deficient in IL-12p35 are not (Hue S, et al. J Exp Med. 2006; 203(11);2473-2483; Yen D, et al. J Clin Invest. 2006;116(5):1310-1316). Preclinical studies in several different animal models of inflammatory bowel disease have demonstrated strong efficacy with IL-23-specific antagonism (Kullberg et al. Langowski et al. Nature. 2006; 442(7101):461-465; Uhlig et al., Immunity 2006;25(2):309-318). Genome-wide association studies revealed that single nucleotide polymorphisms (SNPs) in the gene encoding IL-23R are associated with Crohn's disease (Duerr et al. Science. 2006; 314(5804):1461-1463). Interleukin-23 is elevated in Crohn's disease lesional tissue, while IL-12 is not (Schmidt et al. Inflamm Bowel Dis. 2005;11(1):16-23).

It is contemplated herein that there is a need for new modalities for the treatment of Crohn's disease that specifically target IL-23 without the potential risks associated with inhibition of IL-12. Provided herein are methods for the treatment of Crohn's disease using human monoclonal antibodies that are able to inhibit native human IL-23 while sparing IL-12.

### Summary

Provided herein are methods of treating Crohn's disease in a subject in need thereof comprising administering to the subject an anti-IL-23 antibody in an amount and at an interval of: 15 - 54 mg every 0.5 - 1.5 months; 55 - 149 mg every 1.5 - 4.5 months; 150 - 299 mg every 4 - 8 months; or 300 - 1100 mg every 4 - 12 months. In some embodiments, the amount and interval are: 15 - 21 mg every 0.5 - 1.0 month; 55 - 70 mg every 1.5 - 3.0months; 150 - 260 mg every 4 - 6 months; or 300 - 700 mg every 4 - 8 months. In some embodiments, the amount and interval are: 21 mg every month; 70 mg every 3 months; 210mg every 6 months; or 700 mg every 6 months. In some embodiments, the amount and interval are: 210 mg every 3 months or 700 mg every 3 months. In some embodiments, the amount and interval are: 210 mg every 1 month or 700 mg every 1 month. In some embodiments of the methods, the anti-IL23 antibody is administered IV. In some embodiments of the methods, the anti-IL23 antibody is administered SC. In some embodiments of the methods, the anti-IL-23 antibody is AMG 139.

Also provided herein are methods of treating Crohn's disease in a subject in need thereof comprising administering to the subject an amount of an anti-IL-23 antibody in an amount and at an interval sufficient to achieve and/or maintain a quantity of anti-IL-23 antibody per volume of serum of between 12.5 ng/ml and 1000 ng/ml. In some embodiments, the quantity of an anti-IL-23 antibody per volume of serum is at least 10 ng/ml. In some embodiments, the quantity of an anti-IL-23 antibody per volume of serum is selected from the group consisting of: at least 25 ng/ml; at least 50 ng/ml; at least 60 ng/ml; at least 70 ng/ml; at least 75 ng/ml; and at least 80 ng/ml. In some embodiments, the quantity of an anti-IL-23 antibody per volume of serum is between 85 ng/ml and 100 ng/ml. In some embodiments, the quantity of an anti-IL-23 antibody per volume of serum is between 70 ng/ml and 150 ng/ml. In some embodiments the quantity of an anti-IL-23 antibody per volume of serum is is between 50 ng/ml and 250 ng/ml. In some embodiments, the quantity of an anti-IL-23 antibody per volume of serum is is between 40 ng/ml and 500 ng/ml. In some embodiments, the quantity of an anti-IL-23 antibody per volume of serum is between 25 ng/ml and 750 ng/ml. In some embodiments, the quantity of an anti-IL-23 antibody per volume of serum is between 10 ng/ml and 1,000 ng/ml. In some embodiments of the methods, the anti-IL23 antibody is administered IV. In some embodiments of the methods, the anti-IL23 antibody is administered SC. In some embodiments of the methods, the anti-IL-23 antibody is AMG 139.

### Brief Description of the Drawings

Figure 1 presents the results of the pharmacokinetic analysis of an ascending multiple dose study of AMG 139 in healthy subjects (HS) and Crohn's dsease subjects (CD). The results shown illustrate the mean (± SD) serum AMG 139 concentration-time profiles.
Figure 2 presents the results of individual Crohn's Disease Activity Index (CDAI) Score Profiles after intravenous administration of placebo or 210 mg AMG 139 once every 28 days for a total of 33 doses to two subjects. The results illustrate absolute CDAI score and change from baseline at time points throughout the study. Each arrow represents administration of the investigational product or placebo.
Figure 3 presents the pharmacokinetic structural model used in developing the AMG 139 quantitative population PK model based on data from Example 1.
Figure 4 presents the results of a diagnostic visual predictive check of the AMG 139 population PK model. The results shown illustrate the mean (solid line) and 90% confidence interval (dashed line) AMG 139 concentration-time profile after simulating 1000 clinical trials. Each point represents actual, observed concentrations from subjects.
Figure 5 presents the results of multiple diagnostic visual predictive checks of the AMG 139 population PK model. The results illustrate correlations between observed AMG 139 concentrations and that of population and individual predicted concentrations, as well as the weighted residuals of model fitting between population predicted concentrations and time.
Figure 6 presents the results of a correlation analysis between body weight and PK parameters. The results illustrate a positive correlation in individual CL and V with body weight for the combined population of healthy and PsO subjects.

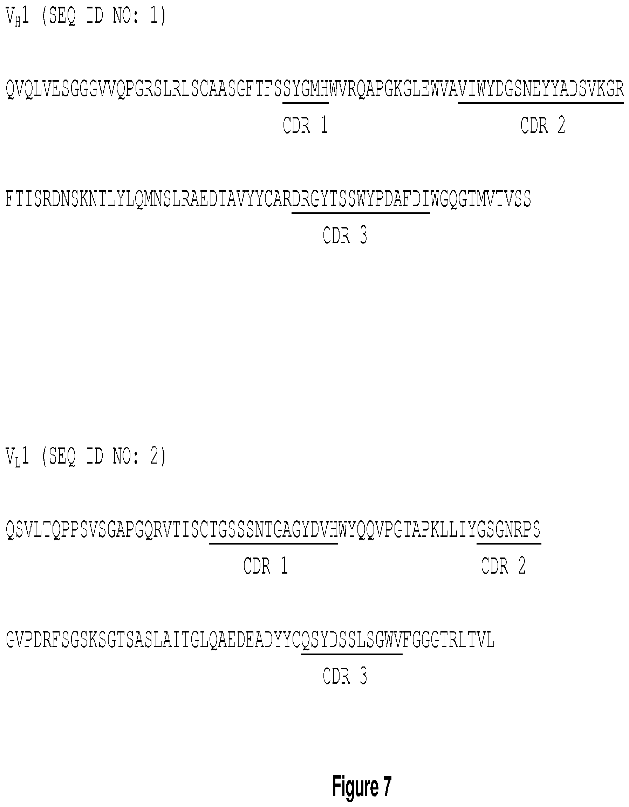

Figure 7 presents the amino acid sequences of AMG 139 heavy and light chain variable regions.

### Detailed Description

Provided herein are methods for treating Crohn's disease in a subject in need thereof comprising administering to the subject an amount of a human monoclonal antibody that specifically binds IL-23. In some embodiments, the anti-IL-23 antibody specifically binds IL-23 but spares IL-12.

The terms "treating", and "treatment" and the like are used herein to generally mean obtaining a desired pharmacological,physiological or therapeutic effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or (c) relieving the disease, i.e., causing regression of the disease and/or its symptoms or conditions. The invention is directed towards treating a patient's suffering from disease related to pathological inflammation. The present invention is involved in preventing, inhibiting, or relieving adverse effects attributed to pathological inflammation over long periods of time and/or are such caused by the physiological responses to inappropriate inflammation present in a biological system over long periods of time.

In one aspect, the present invention provides methods of treating a subject. The method can, for example, have a generally salubrious effect on the subject, *e.g.,* it can increase the subject's expected longevity. Alternatively, the method can, for example, treat, prevent, cure, relieve, or ameliorate ("treat") a disease, disorder, condition, or illness ("a condition"). In one embodiment, the present invention provides a method of treating a condition in a subject comprising administering the pharmaceutical composition comprising an specific antibody to the subject, wherein the condition is treatable by reducing the activity (partially or fully) of IL-23 in the subject. Treating encompasses both therapeutic administration (i.e., administration when signs and symptoms of the disease or condition are apparent) as well prophylactic or maintenance therapy (i.e., administration when the disease or condition is quiescent), as well as treating to induce remission and/or maintain remission. Accordingly, the severity of the disease or condition can be reduced (partially, significantly or completely), or the signs and symptoms can be prevented or delayed (delayed onset, prolonged remission, or quiescence).

Among the conditions to be treated in accordance with the present invention are conditions in which IL-23 is associated with or plays a role in contributing to the underlying disease or disorder or otherwise contributes to a negative symptom. Such conditions include conditions of or associated with the gastrointestinal system, including but not limited to, inflammatory bowel disease (IBD), ulcerative colitis (UC), Crohn's disease (CD), Celiac disease (nontropical Sprue), enteropathy associated with seronegative arthropathies, microscopic or collagenous colitis, lymphatic colitis, eosinophilic gastroenteritis, or pouchitis resulting after proctocolectomy and ileoanal anastomosis.

Disease activity in Crohn's is estimated using the Crohn's Disease Activity Index (CDAI). The CDAI is the oldest and most widely used of several multi-item instruments which have been developed to measure disease activity in Crohn's disease (Sands and Ooi. Inflamm Bowel Dis. 2005;11:133-138). The CDAI is a weighted, composite index of eight items (stool frequency, severity of abdominal pain, degree of general well-being, presence or absence of extraintestinal manifestations or fistula, use or nonuse of antidiarrheal agents, presence or absence of an abdominal mass, hematocrit, and body weight), with scores ranging from approximately 0 to 600, with a higher score indicating more severe disease activity. Typically, patients with scores <150 are considered in remission. Disease is otherwise graded as mild, moderate, or severe with scores of 151-to-219, 220-to-449, and equal or greater than 450, respectively. Induction therapy for patients with mild-to-moderate Crohn's disease typically uses 5-aminosalicylates or sulfasalazine or antimicrobial agents. Disease activity in ulcerative colitis is estimated using scores such as the Mayo score (originally described by Schroeder et al., N Eng J Med. 317:1625; 1987), a composite index of four items (stool frequency, rectal bleeding, endoscopy findings, and physician global assessment) with each item graded semi-quantitatively on a score of 0 to 3 for a maximal total score of 12. The goals of therapy in ulcerative colitis are the same as those cited for Crohn's disease namely induction and maintenance of remission. The therapies available are similar to those for Crohn's disease.

The anti-IL23 antibody may be administered to achieve an improvement in a subject's condition. Improvement may be indicated by a decrease in an index of disease activity, such as CDAI or Mayo score, by amelioration of clinical symptoms or by any other measure of disease activity. In one embodiment, an improvement is considered to be sustained if the subject exhibits the improvement on at least two occasions separated by two to four weeks. In another embodiment, an improvement is considered to be sustained if the subject exhibits the improvement on at least two occasions separated by two to four months; in a further embodiment, an improvement is considered to be sustained if the subject exhibits the improvement on at least two occasions separated by six to twelve months. The degree of improvement generally is determined by a physician, who may make this determination based on signs, symptoms, biopsies, or other test results, and who may also employ questionnaires that are administered to the subject, such as quality-of-life questionnaires developed for a given disease.

The term "efficacy" as used herein in the context of a dosage regimen refers to the effectiveness of a particular treatment regimen. Efficacy can be measured based on change the course of the disease in response to an agent of the present invention. In one embodiment, a therapeutic protein (for example, an anti-IL23 antibody) is administered to the subject in an amount and for a time sufficient to induce an improvement, preferably a sustained improvement, in at least one indicator that reflects the severity of the disorder that is being treated. Various indicators that reflect the extent of the subject's illness, disease or condition may be assessed for determining whether the amount and time of the treatment is sufficient. Such indicators include, for example, clinically recognized indicators of disease severity, symptoms, or manifestations of the disorder in question.

Treatment of a subject with an IL-23 specific antibody may be given in an amount and/or at sufficient interval to achieve and/or maintain a certain quantity of IL-23-specific antibody per volume of serum, using, for example, an assay as described herein. For example, the anti-IL23 antibody is given to achieve at least 25 ng/ml, 50 ng/ml, 60 ng/ml, 70 ng/ml, 75 ng/ml, 80 ng/ml, 85 ng/ml, 90 ng/ml, 95 ng/ml, 100 ng/ml, 150 ng/ml, 200 ng/ml, 500 ng/ml, or 990 ng/ml. In a further embodiment, the anti-IL23 antibody is given to achieve from 12.5 ng/ml to 1000 ng/ml. Those of skill in the art will understand that the amounts given here apply to a full-length antibody or immunoglobulin molecule; if an antigen binding fragment thereof is used, the absolute quantity will differ from that given in a manner that can be calculated based on the molecular weight of the fragment.

Treatment of a subject with an IL-23 specific antibody may be given in an amount and at an interval of 15 - 54 mg every 0.5 - 1.5 months, 55 - 149 mg every 1.5 -4.5 months, 150 - 299mg every 4 -8 months and 300 - 1100 mg every 4 - 12 months. In one embodiment 15 - 21 mg every 0.5 - 1.0 month, 55 - 70 mg every 1.5 -3.0 months, 150 - 260 mg every 4 -6 months and 300 - 700 mg every 4 - 8 months. In another embodiment 21 mg every 1.0 month, 70 mg every 3.0 months, 260 mg every 6 months and 700 mg every 6 months. In one embodiment 260 mg every 3 months and 700 mg every 3 months. In one embodiment 260 mg every 1 month and 700 mg every 1 month.xxx

Subcutaneous or intravenous administration of an anti-IL23 antibody may significantly reduced the symptoms of Crohn's disease as measured by the CDAI scoring system. In some embodiments, administration of an anti-IL23 antibody at the dosages and administration schedules described above may be used to reduce the CDAI score in a patient having Crohn's disease by at least 10, 15, 20, 25, 30, 35, 40, 45, 50, 55, 60, 65, 70, 75, 80, 85, 90, 95, or 100%.

Subcutaneous or intravenous administration of an anti-IL23 antibody may significantly reduced the symptoms of ulcerative colitis as measured by the Mayo scoring system. In some embodiments, administration of an anti-IL23 antibody at the dosages and administration schedules described above may be used to reduce the Mayo core in a patient having ulcerative colitis by at least 10, 15, 20, 25, 30, 35, 40, 45, 50, 55, 60, 65, 70, 75, 80, 85, 90, 95, or 100%.

It is understood that the methods of treating the diseases described herein would administer an effective amount of an anti-IL23 antibody. Depending on the indication to be treated, a therapeutically effective amount is sufficient to cause a reduction in at least one symptom of the targeted pathological condition by at least about 5%, 10%, 15%, 20%, 25%, 30%, 35%, 40%, 45%, 50%, 55%, 60%, 65%, 70%, 75%, 80%, 85%, 90%, 95%, or more, relative to untreated subjects.

Administration and dosage regimens of an anti-IL-23 antibody can be adjusted to provide an effective amount for an optimum therapeutic response. For example, a single bolus can be administered, several divided doses can be administered over time or the dose can be proportionally reduced or increased as indicated by the exigencies of the therapeutic situation. The anti-IL23 antibody may be administered by any suitable technique, including but not limited to, parenterally, topically, or by inhalation. If injected, the pharmaceutical composition can be administered, for example, via intra-articular, intravenous, intramuscular, intralesional, intraperitoneal or cutaneous routes (including intra-, trans- or sub- dermal, and subcutaneous), by bolus injection, or continuous infusion. In some embodiments, the pharmaceutical composition is administered by an intravenous route. In some embodiments the pharmaceutical composition is administered by a subcutaneous route. In further embodiments, the compositions are administered by oral, buccal, rectal, intratracheal, gastric, or intracranial routes. Localized administration, e.g. at a site of disease or injury is contemplated, for example, by enema or suppository for conditions involving the gastrointestinal tract. Also contemplated are transdermal delivery and sustained release from implants. Delivery by inhalation includes, for example, nasal or oral inhalation, use of a nebulizer, inhalation of the antagonist in aerosol form, and the like. Other alternatives include eyedrops; oral preparations including pills, syrups, lozenges or chewing gum; and topical preparations such as lotions, gels, sprays, and ointments, prefilled syringes and autoinjectors.

Advantageously, anti-IL-23 antibodies are administered in the form of a composition comprising one or more additional components such as a physiologically acceptable carrier, excipient or diluent. Optionally, the composition additionally comprises one or more physiologically active agents for combination therapy. A pharmaceutical composition may comprise an anti-IL23 antibody together with one or more substances selected from the group consisting of a buffer, an antioxidant such as ascorbic acid, a low molecular weight polypeptide (such as those having fewer than 10 amino acids), a protein, an amino acid, a carbohydrate such as glucose, sucrose or dextrins, a chelating agent such as EDTA, glutathione, a stabilizer, and an excipient. Neutral buffered saline or saline mixed with conspecific serum albumin are examples of appropriate diluents. In accordance with appropriate industry standards, preservatives such as benzyl alcohol may also be added. The composition may be formulated as a lyophilizate using appropriate excipient solutions (e.g., sucrose) as diluents. The IL-23 antibody can be provided at a concentration of 50 to 200 mg/ml. Exemplary formulations useful for the present invention are those that include a glutamic acid, citric acid or acetic acid buffer at an appropriate pH, from 4.5 to 5.2, an excipient such as sucrose, glycine, proline, glycerol, and/or sorbitol at an appropriate concentration such as 1 to 20% (w/v), and a surfactant such as a non-ionic surfactant like polysorbate (polysorbate 20 or 80) or poloxamers (poloxamer 1888) at an appropriate concentration of 0.001% - 0.1% (w/v). Such formulations are disclosed in US Patent No. 6171586 and WIPO Published Applications Nos: WO20100027766 and WO2011088120. In some embodiments, the formulations comprise sodium acetate, sucrose and polysorbate 20. In some embodiments, the formulations comprise 70 mg/mL AMG 139, 10 mM sodium acetate, 9% (w/v) sucrose and 0.004% (w/v) polysorbate 20, at pH 5.2. Suitable components are nontoxic to recipients at the dosages and concentrations employed. Further examples of components that may be employed in pharmaceutical formulations are presented in any Remington's Pharmaceutical Sciences including the 21st Ed. (2005), Mack Publishing Company, Easton, PA.

Kits for use by medical practitioners include an anti-IL-23 antibody and a label or other instructions for use in treating any of the conditions discussed herein. In one embodiment, the kit includes a sterile preparation of one or more IL-23 binding antigen binding proteins, which may be in the form of a composition as disclosed above, and may be in one or more vials.

Particular embodiments of methods of the invention involve the use of an anti-IL23 antibody and one or more additional IL-23 antagonists, as described in US Patents US 7,491,391; US 7,807,414; US7,872,102; US7,807,160; US8362212; US7,935,344; US 7,790,862; US2012282269; US Published Patent Applications US 2009-0123479; US 20120128689; and US2012264917 and WIPO Publications WO1999/05280, WO2007/0244846, WO2007/027714, WO 2007/076524, WO2007/147019, WO2008/103473, WO 2008/103432, WO2009/043933, WO2009/082624 WO 12/009760. Also included are p40 antibodies such as ustekinumab (Stelara®), and briakinumab.

Additional types of combinations may be employed with the IL-23antagonists described herein. Examples include using combinations of an anti-IL23 antibody and one or more other therapeutic moiety having anti-inflammatory properties (for example, non-steroidal anti-inflammatory agents, steroids, and/or immunomodulators), or of an anti-IL23 antibody and one or more other treatments (*e.g.*, surgery, ultrasound, or treatment effective to reduce inflammation). Useful agents that may be combined with an anti-IL23 antibody include those used to treat, for example, Crohn's disease or ulcerative colitis, such as aminosalicylate (for example, mesalamine or substances that are metabolized to mesalamine, including, for example, Asacol®, salofalk, Pentasa®, Dipentum®, colazide, Lialda® and Rowasa®), corticosteroids/glucocorticoids (including prednisolone methasulfobenzoate, tixocortol pivalate, fluticasone propionate, beclomethasone dipropionate, and budesonide), antibiotics such as metronidazole or ciprofloxacin (or other antibiotics useful for treating, for example, patients afflicted with fistulas), and immunosupporessives such as azathioprine (for example, Imuran® and Azasan®), 6-mercaptopurine (for example, Purinethol®), methotrexate (for example, Trexall®, Rheumatrex®), tacrolimus (for example, Prograf®) and cyclosporine (for example, Gengraf®, Neoral®, and Sandimmune®). Such agent(s) may be administered orally or by another route, for example via suppository or enema, at dosages and intervals that are known in the art and described in the prescribing information.

Furthermore, IL-23 antibodies or antibody derivatives, or the aforesaid combinations, can be used in conjunction with one or more molecules or other treatments, wherein the other molecule(s) and/or treatment(s) do not directly bind to or affect IL-23, but which combination is effective for treating or preventing the condition being treated. For example, an anti-IL23 antibody can be used in combination with probiotic therapy, or other therapy used to restore or maintain normal gut flora, including gut flora transplant. In one embodiment, one or more of the molecule(s) and/or treatment(s) treats or prevents a condition that is caused by one or more of the other molecule(s) or treatment(s) in the course of therapy, *e.g.,* nausea, fatigue, alopecia, cachexia, insomnia, *etc.* Such agent(s) or therapies may be administered by routes, and at dosages and intervals, that are known in the art and described in the prescribing information.

Additional supportive therapies are included in possible combination treatment with IL-23 antibodies; such supportive therapies as (without limitation), analgesics, and anticholinergic and antidiarrheal agents. Combining such supportive therapies can be useful in the beginning of a treatment regimen in reducing a patient's symptoms and improving their quality of life. Supportive therapies include administering oral iron, folate, and vitamin B₁₂. Antidiarrheal agents include, but are not limited to diphenoxylate, codeine, loperamide, and anticholinergics (or pharmacological equivalents thereof), which can be administered to patients with mild disease to reduce the frequency of bowel movements and relive rectal urgency. Cholestyramine can be used in patients to prevent bile salt-induced colonic secretion in patients who have already undergone limited ileocolic resections. Anticholinergic agents include, but are not limited to, clidinium bromide, dicyclomine hydrochloride, tincture of belladonna and the like, and are useful to reduce abdominal cramps, pain and rectal urgency. Supportive or therapies may be administered by routes, and at dosages and intervals, that are known in the art and described in the prescribing information

In every case where a combination of molecules and/or other treatments is used, the individual molecule(s) and/or treatment(s) can be administered in any order, over any length of time, which is effective, *e.g.,* simultaneously, consecutively, or alternately. In one embodiment, the method of treatment comprises completing a first course of treatment with one molecule or other treatment before beginning a second course of treatment. The length of time between the end of the first course of treatment and beginning of the second course of treatment can be any length of time that allows the total course of therapy to be effective, *e.g.,* seconds, minutes, hours, days, weeks, months, or even years.

The terms "polypeptide" or "protein" means a macromolecule having the amino acid sequence of a native protein, that is, a protein produced by a naturally-occurring and non-recombinant cell; or it is produced by a genetically-engineered or recombinant cell, and comprise molecules having the amino acid sequence of the native protein, or molecules having one or more deletions from, insertions to, and/or substitutions of the amino acid residues of the native sequence. The term also includes amino acid polymers in which one or more amino acids are chemical analogs of a corresponding naturally-occurring amino acid and polymers. The terms "polypeptide" and "protein" encompass IL-23 antibodies and sequences that have one or more deletions from, additions to, and/or substitutions of the amino acid residues of the antigen binding protein sequence. The term "polypeptide fragment" refers to a polypeptide that has an amino-terminal deletion, a carboxyl-terminal deletion, and/or an internal deletion as compared with the full-length native protein. Such fragments may also contain modified amino acids as compared with the native protein. In certain embodiments, fragments are about five to 500 amino acids long. For example, fragments may be at least 5, 6, 7, 8, 9, 10, 11, 12, 13, 14, 15, 16, 17, 18, 19, 20, 50, 70, 100, 110, 150, 200, 250, 300, 350, 400, or 450 amino acids long. Useful polypeptide fragments include immunologically functional fragments of antibodies, including binding domains. In the case of an anti-IL23 antibody, useful fragments include but are not limited to one or more CDR regions, a variable domain of a heavy or light chain, a portion of an antibody chain, a portion of a variable region including less than three CDRs, and the like.

The term "isolated protein" refers to a protein, such as an antigen binding protein (an example of which could be an antibody), that is purified from proteins or polypeptides or other contaminants that would interfere with its therapeutic, diagnostic, prophylactic, research or other use. As used herein, "substantially pure" means that the described species of molecule is the predominant species present, that is, on a molar basis it is more abundant than any other individual species in the same mixture. In certain embodiments, a substantially pure molecule is a composition wherein the object species comprises at least 50% (on a molar basis) of all macromolecular species present. In other embodiments, a substantially pure composition will comprise at least 80%, 85%, 90%, 95%, or 99% of all macromolecular species present in the composition. In certain embodiments, an essentially homogeneous substance has been purified to such a degree that contaminating species cannot be detected in the composition by conventional detection methods and thus the composition consists of a single detectable macromolecular species.

A "variant" of a polypeptide (e.g., an antigen binding protein such as an antibody) comprises an amino acid sequence wherein one or more amino acid residues are inserted into, deleted from and/or substituted into the amino acid sequence relative to another polypeptide sequence. Variants include fusion proteins. A "derivative" of a polypeptide is a polypeptide that has been chemically modified in some manner distinct from insertion, deletion, or substitution variants, e.g., via conjugation to another chemical moiety.

The terms "naturally occurring" or "native" as used throughout the specification in connection with biological materials such as polypeptides, nucleic acids, host cells, and the like, refers to materials which are found in nature, such as native human IL-23. In certain aspects, recombinant antigen binding proteins that bind native IL-23 are provided. In this context, a "recombinant protein" is a protein made using recombinant techniques, i.e., through the expression of a recombinant nucleic acid as described herein. Methods and techniques for the production of recombinant proteins are well known in the art.

The term "antibody" refers to an intact immunoglobulin of any isotype, or a fragment thereof that can compete with the intact antibody for specific binding to the target antigen, and includes, for instance, chimeric, humanized, fully human, and bispecific antibodies. An antibody as such is a species of an antigen binding protein. Unless otherwise indicated, the term "antibody" includes, in addition to antibodies comprising two full-length heavy chains and two full-length light chains, derivatives, variants, fragments, and muteins thereof, examples of which are described below. An intact antibody generally will comprise at least two full-length heavy chains and two full-length light chains, but in some instances may include fewer chains such as antibodies naturally occurring in camelids which may comprise only heavy chains. Antibodies may be derived solely from a single source, or may be "chimeric," that is, different portions of the antibody may be derived from two different antibodies as described further below. The antigen binding proteins, antibodies, or binding fragments may be produced in hybridomas, by recombinant DNA techniques, or by enzymatic or chemical cleavage of intact antibodies.

The term "functional fragment" (or simply "fragment") of an antibody or immunoglobulin chain (heavy or light chain), as used herein, is an antigen binding protein comprising a portion (regardless of how that portion is obtained or synthesized) of an antibody that lacks at least some of the amino acids present in a full-length chain but which is capable of specifically binding to an antigen. Such fragments are biologically active in that they bind specifically to the target antigen and can compete with other antigen binding proteins, including intact antibodies, for specific binding to a given epitope. In one aspect, such a fragment will retain at least one CDR present in the full-length light or heavy chain, and in some embodiments will comprise a single heavy chain and/or light chain or portion thereof. These biologically active fragments may be produced by recombinant DNA techniques, or may be produced by enzymatic or chemical cleavage of antigen binding proteins, including intact antibodies. Fragments include, but are not limited to, immunologically functional fragments such as Fab, Fab', F(ab')2, Fv, domain antibodies and single-chain antibodies, and may be derived from any mammalian source, including but not limited to human, mouse, rat, camelid or rabbit. It is contemplated further that a functional portion of the antigen binding proteins disclosed herein, for example, one or more CDRs, could be covalently bound to a second protein or to a small molecule to create a therapeutic agent directed to a particular target in the body, possessing bifunctional therapeutic properties, or having a prolonged serum half-life.

An "antigen binding protein" as used herein means a protein that specifically binds a specified target antigen; the antigen as provided herein is IL-23, particularly human IL-23, including native human IL-23. Antigen binding proteins as provided herein interact with at least a portion of the unique p19 subunit of IL-23, detectably binding IL-23; but do not bind with any significance to IL-12 (e.g., the p40 and/or the p35 subunits of IL-12), thus "sparing IL-12". As a consequence, the antigen binding proteins provided herein are capable of impacting IL-23 activity without the potential risks that inhibition of IL-12 or the shared p40 subunit might incur. The antigen binding proteins may impact the ability of IL-23 to interact with its receptor, for example by impacting binding to the receptor, such as by interfering with receptor association. In particular, such antigen binding proteins totally or partially reduce, inhibit, interfere with or modulate one or more biological activities of IL-23. Such inhibition or neutralization disrupts a biological response in the presence of the antigen binding protein compared to the response in the absence of the antigen binding protein and can be determined using assays known in the art and described herein. Antigen binding proteins provided herein inhibit IL-23-induced proinflammatory cytokine production, for example IL-23-induced IL-22 production in whole blood cells and IL-23-induced IFNγ expression in NK and whole blood cells. Reduction of biological activity can be about 20%, 30%, 40%, 50%, 60%, 70%, 80%, 85%, 90%, 91%, 92%, 93%, 94%,95%, 96%, 97% 98%, 99% or more.

Certain antigen binding proteins described herein are antibodies, or are derived from antibodies. Such antigen binding proteins include, but are not limited to, monoclonal antibodies, bispecific antibodies, minibodies, domain antibodies, synthetic antibodies, antibody mimetics, chimeric antibodies, humanized antibodies, human antibodies, antibody fusions, antibody conjugates, single chain antibodies, and fragments thereof, respectively. In some instances, the antigen binding protein is an immunological fragment of an antibody (e.g., a Fab, a Fab', a F(ab')2, or a scFv). Certain antigen binding proteins that are provided may comprise one or more CDRs as described herein (e.g., 1, 2, 3, 4, 5, 6 or more CDRs). In some instances, the antigen binding protein comprises (a) a polypeptide structure and (b) one or more CDRs that are inserted into and/or joined to the polypeptide structure. The polypeptide structure can take a variety of different forms. For example, it can be, or comprise, the framework of a naturally occurring antibody, or fragment or variant thereof, or may be completely synthetic in nature. Examples of various polypeptide structures are further described below.

An antigen binding protein of the invention is said to "specifically bind" its target antigen when the dissociation equilibrium constant (KD) is ≤ 10⁻⁸ M. The antigen binding protein specifically binds antigen with "high affinity" when the KD is ≤ 5 x 10⁻⁹ M, and with "very high affinity" when the the KD is ≤ 5 x 10⁻¹⁰ M. In one embodiment the antigen binding protein will bind to human IL-23 with a KD of ≤ 5 x 10⁻¹² M, and in yet another embodiment it will bind with a KD ≤ 5 x 10⁻¹³ M. In another embodiment of the invention, the antigen binding protein has a KD of ≤ 5 x 10⁻¹² M and an Koff of about ≤5x10⁻⁶ 1/s. In another embodiment, the Koff is ≤ 5x10⁻⁷1/s.

In embodiments where the antigen binding protein is used for therapeutic applications, an antigen binding protein can reduce, inhibit, interfere with or modulate one or more biological activities of IL-23, such inducing production of proinflammatory cytokines. IL-23 has many distinct biological effects, which can be measured in many different assays in different cell types; examples of such assays and known see for example US Patent Application No: US 2013-0004501, the disclosure of which is incorporated by reference herein Exemplary IL-23 antibodies are disclosed US Patent Application No: US 2013-0004501.

As used herein, "AMG 139" refers to an intact AMG 139 immunoglobulin or to an antigen binding portion thereof that competes with the intact antibody for specific binding, unless otherwise specified. AMG 139 also includes antibodies (or fragments thereof) that are identical or similar to AMG 139 in amino acid sequence, particularly in the variable regions, or in the CDRs thereof (however, variations in the constant regions are also contemplated). For example, a useful AMG 139 polypeptide has an amino acid sequence that is 85%, 90%, 92%, 95%, 98%, 99% or 100% identical to that of an AMG 139 polypeptide disclosed herein. In another embodiment, a useful polypeptide is between 80% and 100% identical to AMG 139.

AMG139 is a human antibody that specifically recognizes the native human IL-23 heterodimer, but does not bind with any significance to the human IL-12 heterodimer. AMG139 inhibits IL-23-induced proinflammatory cytokine production, for example IL-23-induced IL-22 production in whole blood cells and IL-23-induced IFNγ expression in NK and whole blood cells. In some embodiments, AMG 139 is an isolated, IL-23 specific antigen binding protein having a heavy chain variable region comprising CDR1, CDR2 and CDR3 from NO:1, and a light chain variable region comprising CDR1, CDR2 and CDR3 from SEQ ID NO:2. In some embodiments, AMG 139 is an isolated, IL-23 specific antigen binding protein wherein the heavy chain variable region is at least 90% identical to SEQ ID NO:1, and the light chain variable region is at least 90% identical to CDR1, CDR2 and CDR3 from SEQ ID NO:2. See, WO 2011/056600 published May 11, 2011.

Where a range of values is provided, it is understood that each intervening value (to the tenth of the unit of the lower limit unless the context clearly dictates otherwise) between the upper and lower limit of that range, and any other stated or intervening value or smaller range, in that stated range is encompassed within the invention. The upper and lower limits of smaller ranges may independently be included in the smaller range, subject to any specifically excluded limit in the stated range. Where the stated range includes one or both of the limits, ranges excluding either both of those included limits are also included in the invention.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well known and commonly used in the art. Standard techniques can be used for chemical syntheses, chemical analyses, pharmaceutical preparation, formulation, and delivery, and treatment of patients.

All patents and other publications identified are expressly incorporated herein by reference in their entirety for the purpose of describing and disclosing, for example, the methodologies described in such publications that might be used in connection with information described herein.

The following examples, both actual and prophetic, are provided for the purpose of illustrating specific embodiments or features of the instant invention and do not limit its scope.

### Example 1

This example describes a Phase 1, randomized, double-blind, placebo-controlled, ascending multiple dose study to evaluate the safety, tolerability, pharmacokinetics (PK) and pharmacodynamics (PD) of an anti-IL-23 antibody (AMG 139) in healthy subjects (HS) and subjects with mild to severe Crohn's disease; (Clinicaltrials.gov Identifer NCT01258205).

### Part A

Part A (healthy subjects) consisted of 5 dose cohorts; 4 intravenous (IV); 1 hour infusion, cohorts and 1 subcutaneous (SC) cohort. Part A followed a randomized, multiple-dose (3 IV or SC doses on Days 1, 29 and 57), double-blind, placebo-controlled sequential dose-escalation study design. Within each cohort, 8 subjects were enrolled and assigned the investigational product (AMG 139 or placebo) in a 3 to 1 randomization ration, such that 6 subjects received AMG 139 and 2 received placebo. See Table 1 for dose levels for each dose cohort.

**Table 1. Dosage and Administration Route for Each Cohort**

| Cohorts | Dosage (mg) | Route | Number of Subjects (AMG 139:placebo) |
|---|---|---|---|
| PART A | | | Healthy Subjects |
| Cohort A1 | 70 | IV | 8 (6:2) |
| Cohort A2 | 210 | IV | 8 (6:2) |
| Cohort A3 | 420 | IV | 8 (6:2) |
| Cohort A4 | 210 | SC | 8 (6:2) |
| Cohort A5 | 700 | IV | 8 (6:2) |

### Serum Concentrations for Pharmacokinetic Analysis

Blood samples for determination of AMG 139 serum concentration were obtained at the following timepoints:
Part A: Days 1 [pre-dose and 0.5 (IV cohorts only), 1, and 4-hour post dose], 4, 8, 15, 29 (pre-dose), 57 [pre-dose and 0.5 (IV cohorts only), 1, and 4-hour post dose], 60, 64, 71, 85, 113, 141, 169, 197, 225 and 253 End of Study (EOS).

For IV dosing cohorts (A1 to A3 and A5), the 30 minutes PK blood samples were drawn from the opposite arm used for infusion and 1-hour PK blood samples were collected at the completion of the IV infusion and the IV flush with 5% dextrose.

To measure the amount of AMG 139 in serum from a subject, capture antibody (mouse anti-AMG 139 1F2 mAb) was passively adsorbed to Multi-Array® 96-well HighBind microplate wells (Meso Scale Discovery). The microplate wells were blocked with Blocker™ BLOTTO buffer after removing excess capture antibody. Standards and quality control samples, prepared by spiking known quantities of AMG 139 into 100% normal human serum pool, were loaded into the microplate wells after pre-treating with a dilution factor of 100 in Blocker™ BLOTTO buffer, as are samples to be tested and matrix blank. Any AMG 139 in the samples was captured by the immobilized capture antibody. Unbound material was removed by washing the microplate wells. Following washing, SULFO-TAGTM conjugated detection antibody (anti-AMG 139 1A4.1 mAb) was added to the microplate wells to bind captured AMG 139. Unbound SULFO-TAGTM conjugated capture antibody was removed by washing the microplate wells.

Following this washing, Read Buffer T (Meso Scale Discovery) was added to aid in the detection of bound SULFO-TAGTM conjugated detection antibody. When the microplate is electrically stimulated, the SULFO-TAGTM label, in the presence of the co-reactant tripropylamine (TPA) in the read buffer, emits light at 620 nm. The quantity of light emitted is proportional to the amount of AMG 139 bound by the capture antibody in the initial step. Light emission was detected using an appropriate plate reader; for example, a Sector Imager 6000 equipped with Discovery Workbench software. Data were reduced using Watson Laboratory Information Management System data reduction package using a 5PL (autoestimate) (5-parameter logistic) regression model with a weighting factor of 1/Y2. The amount of AMG 139 in a given serum sample was determined by comparison to the standard curve formed by the standards and quality control samples.

### Blood Collection for Anti-AMG 139 Antibody Analysis

Blood samples for antibody determination were obtained by the PI or designee at the following timepoints:
Part A: Days 1 (pre-dose), 29 (pre-dose), 57 (pre-dose), 85, 113, 141, 197 and 253 (EOS)

Collection of follow up samples will be done approximately every 3 months (from date of positive result) from all subjects testing neutralizing antibody positive at end of study (EOS), regardless of baseline result. Subjects will be followed until: 1) the sample tests negative, 2) the subject withdraws consent from study, 3) if it is established that the subject did not receive the investigational product or 4) up to 1 year (+/-28 day window) after the date the positive result was obtained, whichever occurs first. Follow-up samples are not mandated for subjects who develop binding, but not neutralizing antibodies.

### Whole Blood Collection for TBNK Cell Count

For all subjects, 5 mL of blood for the enumeration of T-Cell, B-Cell and natural killer cells from whole blood was obtained by the PI or designee at the following time points:
Part A: Baseline (Day -1 or Day 1 pre-dose), and Days 8, 15, 29 (pre-dose), 43, 57 (pre-dose), 71, 113, 141, 169, 197, 225 and 253 (EOS).

Binding antibodies to AMG 139 were detected by an assay utilizing a electrochemiluminescence (ECL) MSD (Meso Scale Discovery) technology platform, which is based on multivalent characteristics of antibody binding. The testing strategy involved a tiered two-assay approach consisting of a screening assay and a specificity assay. Samples with signal to noise ratio (S/N) greater than assay cut point in the screening assay were further tested in the specificity assay by incubating the sample with excess AMG 139 prior to testing.

To enable dissociation of antibody complexes, acid treatment of samples was performed prior to analysis. Acid-treated serum samples and controls were added to a solution consisting of equal parts of biotinylated-AMG 139 (B-AMG 139) and ruthenylated-AMG 139 (Ru-AMG 139) in 1 M Tris, pH 9.5, and are incubated at ambient temperature to allow for anti-AMG 139 antibodies to bind both a B-AMG 139 molecule and a Ru-AMG 139 molecule, thereby forming a complex.

Following the incubation, all samples and controls are transferred to a washed streptavidin-coated standard bind MSD plate blocked with bovine serum albumin and incubated at ambient temperature to allow for the capture of B-AMG 139 and formed complexes on the streptavidin surface. The plate wells are washed and a solution of MSD read buffer containing tripropylamine is added. The plate is read on the MSD Sector Imager 6000 plate reader. Within the instrument, ruthenium participates in an electrochemiluminescent reaction that is triggered when the voltage was applied. The complexes containing the Ru-AMG 139 that are captured on the wells of the plate result in an ECL signal proportionate to the concentration of anti-AMG 139 antibodies in the sample.

### Part B

Subjects with mild to severe Crohn's disease will consist of two IV cohorts. Part B will follow a randomized, multiple-dose (3 IV doses on Days 1, 29 and 57), double-blind, placebo-controlled study design. For the two Crohn's disease cohorts (B1 and B2), 4 subjects will be enrolled in each cohort and will be assigned investigational product (AMG139 or placebo) in a 3 to 1 randomization ratio, such that 3 subjects will receive AMG139 IV infusion, and 1 subject will receive AMG139 placebo IV infusion.

**Table 2. Dosage and Administration Route for Each Cohort**

| Cohorts | Dosage (mg) | Route | Number of Subjects (AMG 139:placebo) |
|---|---|---|---|
| PART B | | | Healthy Subjects |
| Cohort B1 | 210 | IV | 4 (3:1) |
| Cohort B2 | 700 | IV | 4 (3:1) |

### Serum Concentrations for Pharmacokinetic Analysis

Blood samples for determination of AMG 139 serum concentration are to be obtained at the following timepoints:
Part B: Days 1 [pre-dose and 0.5 (IV cohorts only), 1, and 4-hour post dose], 4, 8, 15, 29 (pre-dose), 57 [pre-dose and 0.5 (IV cohorts only), 1, and 4-hour post dose], 60, 64, 71, 85, 113, 141, 169, 197, 225 and 253 (EOS).

For IV dosing cohorts (B1 and B2), the 30 minutes PK blood samples will be drawn from the opposite arm used for infusion and 1-hour PK blood samples will be collected at the completion of the IV infusion and the IV flush with 5% dextrose.

### Blood Collection for Anti-AMG 139 Antibody Analysis

Blood samples for antibody determination will be obtained by the PI or designee at the following timepoints:
Part B: Days 1 (pre-dose), 29 (pre-dose), 57 (pre-dose), 85, 113, 141, 197 and 253 (EOS)

Follow-up samples will be collected approximately every 3 months (from date of positive result) from all subjects testing neutralizing antibody positive at end of study (EOS), regardless of baseline result. Subjects will be followed until: 1) the sample tests negative, 2) the subject withdraws consent from study, 3) if it is established that the subject did not receive the investigational product or 4) up to 1 year (+/-28 day window) after the date the positive result was obtained, whichever occurs first. Follow-up samples are not mandated for subjects who develop binding, but not neutralizing antibodies.

### Whole Blood Collection for TBNK Cell Count

For all subjects, 5 mL of blood for the enumeration of T-Cell, B-Cell and natural killer cells from whole blood will be obtained by the PI or designee at the following time points:
Part B: Screening, Baseline (Day -3 thru -1 or Day 1 pre-dose), and Days 8, 15, 29 (pre-dose), 43, 57 (pre-dose), 71, 113, 141, 169, 197, 225 and 253(EOS).

### Blood Collection for C-reactive protein (CRP)

Blood for determining CRP levels in CD subjects will be obtained by the PI or designee at the following time points: Screening, Baseline (Day -3 thru -1), and Days 29 (pre-dose), 43, 57 (pre-dose), 85, 113, 141, 169, 197, 225 and 253(EOS).

### Crohn's Disease Activity Index (CDAI)

The CDAI will be calculated from entries in patient diary, medical history, and hematology lab by a gastroenterologist or a gastroenterologist-trained health care provider at the following timepoints:
Screening, baseline (Day -3 to Day -1), and Days 15, 29 (pre-dose), 43, 57 (pre-dose), 85, 113, 141, 169, 197, 225 and 253(EOS).

CDAI scores range from 0 to 600, with higher scores indicating greater disease activity. Patients with scores of < 150, 150 to 219, 220 to 450 are considered to be in remission, mild disease and moderate to severe disease, whereas those with scores of > 450 have very severe disease (Buxtonet al. Value Health. 2007;10:214-220). The values of the subjective and objective items for the CDAI will be collected.

### Endoscopic Score

The Simple Endoscopic Score for Crohn's Disease (SES-CD) will be used as a measurement of endoscopic disease activity in Crohn's disease. The SES-CD will be performed by a gastroenterologist at the following timepoints: Screening (Day -28 to Day -8) and Day 85

### Mucosal Biopsies

Colonic mucosal biopsies will be completed through colonoscopy. The mucosal biopses will be performed at the following time points: Screening (Day -28 to Day -8) and Day 85.

The mucosal biopsies will be evaluated for histopathology, immunohistochemistry, and mRNA transcript profiling.

### Results

As of the data cutoff date, 40 subjects had been randomized and received at least one dose of investigational product (AMG 139 or placebo) in Part A according to protocol, and enrollment in Part A was complete. Twenty of the 40 subjects had completed the study, 18 were ongoing, and 2 had discontinued study early for reasons of lost to follow-up full and consent withdrawn.

As of the data cutoff date, 2 subjects had additionally been randomized and received investigational product (AMG 139 or placebo) in Cohort B1 of Part B. One of these 2 subjects had completed study; no treatment emergent adverse events (TESAEs) or deaths had been reported, and no subjects had discontinued the study because of a TEAE.

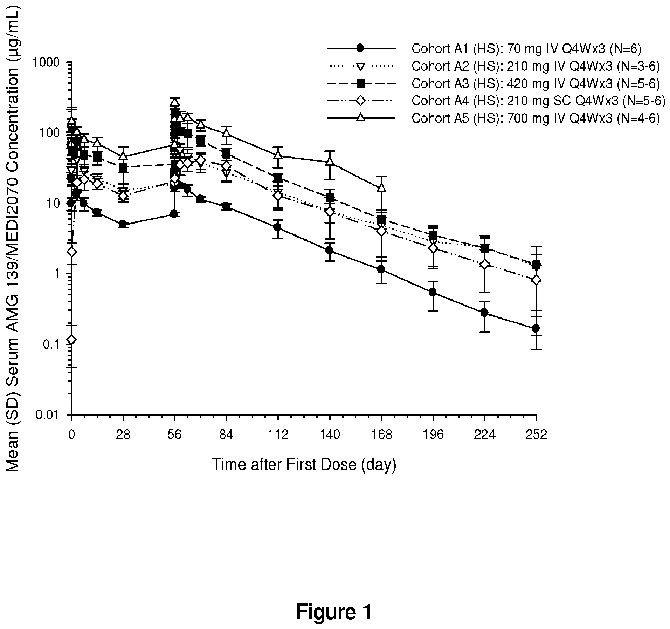

In Part A, healthy subjects received AMG 139 in 3 monthly doses of 70 mg IV (Cohort A1), 210 mg IV (Cohort A2), 420 mg IV (Cohort A3), 210 mg SC (Cohort A4), and 700 mg IV (Cohort A5). AMG 139 concentration versus time profiles in healthy subjects (n = 24) exhibited linear PK (Figure 1), as indicated by serum AMG 139 exposure (Cₘₐₓ and AUC_{τ}) that increased approximately dose proportionally across all IV doses tested after both Dose 1 and Dose 3 (Table 2). The median Tₘₐₓ values at 210 mg SC ranged from 7 to 14 days after AMG 139 administration (Table 2). Relative bioavailabilities after single or 3 doses of 210 mg SC were estimated to be 64.3% and 86.4%, respectively. Group mean estimates of terminal half-life after SC or IV administration across all dose levels ranged from 28.6 to 36.7 days, which is similar to that observed in the ascending, single-dose Study 20080767. AMG 139 was estimated to accumulate between 1.51- to 2.15-fold after 3 doses under the tested dose levels of 70 to 700 mg IV and 210 mg SC in healthy subjects. To maintain blinding PK data could be presented from the subjects with CD (Part B) as of the data cutoff date.

Anti-drug antibody testing has been performed on all samples collected to the data cutoff date, and no subjects had developed anti drug antibodies. Therefore, the potential effects of immunogenicity on AMG 139 disposition could not be assessed.

**Table 2 Descriptive Statistics for AMG 139 PK Parameters after 3 Monthly Doses in Healthy Subjects**

| **Dose Number** | **Route** | **Dose** | **Cₘₐₓ (µg/mL)^{a}** | **tₘₐₓ (day)^{a}** | **AUCτ (day^{∗}µg/mL)^{a}** | **AR** | **t_{1/2,z} (day)** |
|---|---|---|---|---|---|---|---|
| Dose 1 | IV | 70 mg IV | 22.9 | 0.10 | 244 | N/A | N/A |
| | | | (4.37) | (0.04-0.17) | (30.3) | N/A | N/A |
| | | 210 mg IV | 77.3 | 0.042 | 742 | N/A | N/A |
| | | | (16.3) | (0.042-3.0) | (129) | N/A | N/A |
| | | 420 mg IV | 138 | 0.17 | 1400 | N/A | N/A |
| | | | (36.8) | (0.04-0.17) | (284) | N/A | N/A |
| | | 700 mg IV | 164 | 0.10 | 2060 | N/A | N/A |
| | | | (52.0) | (0.042-7.0 | (345) | N/A | N/A |
| | SC | 210 mg SC | 22.4 | 7.0 | 477 | N/A | N/A |
| | | | (6.04) | (3.0-14) | (96.9) | N/A | N/A |
| Dose 3 | IV | 70 mg IV | 27.2 | 0.17 | 367 | 1.51 | 28.6 |
| | | | (4.20) | (0.042-0.17) | (25.3) | (0.133) | (3.16) |
| | | 210 mg IV | 87.4 | 0.10 | 1180 | 1.61 | 36.7 |
| | | | (13.9) | (0.042-0.17) | (263) | (0.334) | (21.4) |
| | | 420 mg IV | 185 | 0.17 | 2330 | 1.68 | 32.7 |
| | | | (60.5) | (0.042-0.17) | (427) | (0.204) | (4.53) |
| | | 700 mg IV | 278 | 0.042 | 3830 | 1.88 | 28.8 |
| | | | (47.3) | (0.042-0.17) | (632) | (0.338) | (6.00) |
| | SC | 210 mg SC | 44.7 | 14.0 | 1020 | 2.15 | 34.3 |
| | | | (9.12) | (3.0-28) | (231) | (0.258) | (5.17) |

| | | | | | | | |
|---|---|---|---|---|---|---|---|
| AR = accumulation ratio or AUC_{τ},dose 3/AUC_{τ}, dose 1; AUC_{τ} = area under the concentration-time curve within the reference dosing intervals; Cₘₐₓ = maximum observed concentration post the reference dose; IV = intravenous(ly); N/A = not applicable; SC = subcutaneous(ly); t_{1/2,z} = elimination half-life; tₘₐₓ = time to maximum observed concentration within the reference dosing interval ^{a}PK parameters are reported as mean (SD) with 3 significant figures except for tₘₐₓ which is reported as median (min-max) rounded to 2 significant figures. | | | | | | | |

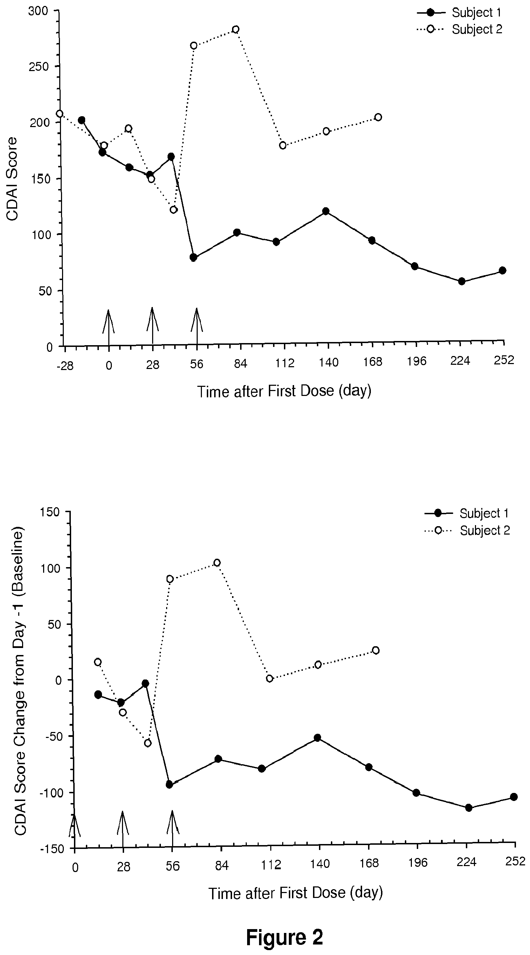

Efficacy was assessed in the two CD subjects of Part B, Cohort B1 using the CD activity index (CDAI) score. The blinded data shows a possible time-dependent difference in CDAI score between the two available CD subjects receiving either placebo or IV administration of 210 mg of AMG 139 (Figure 2).

### Example 2

A quantitative population pharmacokinetics (pop PK) model for AMG 139 was established to simulate the PK of future dosing regimens, as well as incorporation with a quantitative PK/pharmacodynamic model for simulating AMG 139 efficacy. The pop PK model was based on healthy subject and PsO patient data from a Phase 1a FIH (Clinicaltrials.gov Identifer NCT01094093).

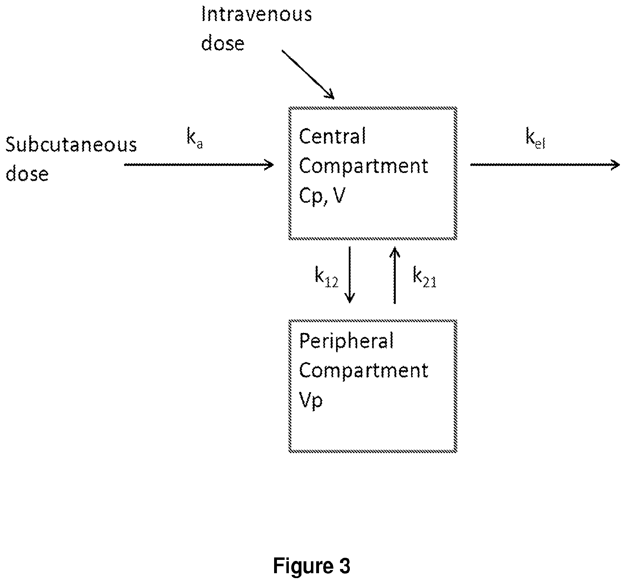

Pop PK modeling of subcutaneous (SC; 7, 21, 70, or 210 mg) or intravenous (IV; 210, 420, or 700 mg) doses was performed with NONMEM v7.2. Data analysis used individual PK data fit simultaneously to a structural two-compartment model with first-order elimination from the central compartment and first-order absorption from a depot compartment (Figure 3). The inter-subject variability parameters and residual error model were varied to obtain the lowest objective function. Body weight and disease were explored as potential PK covariates.

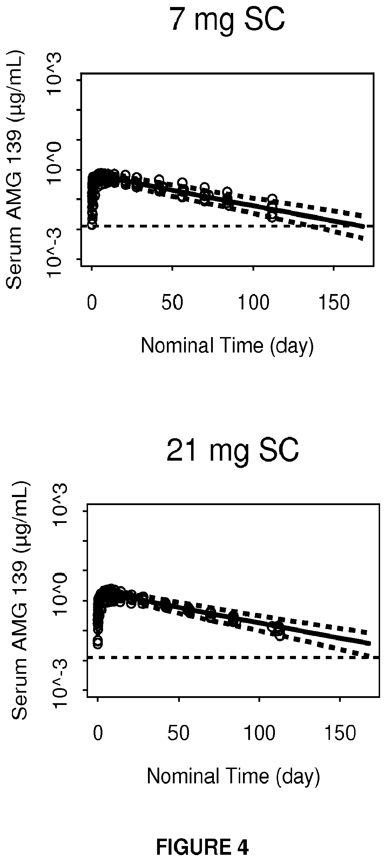

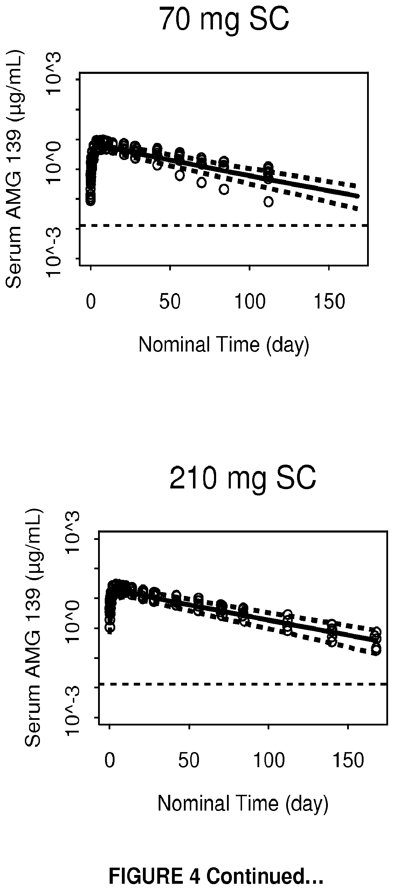

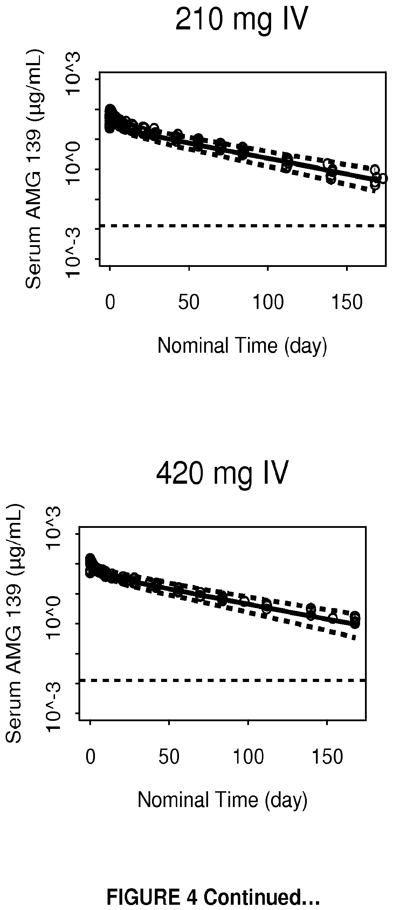

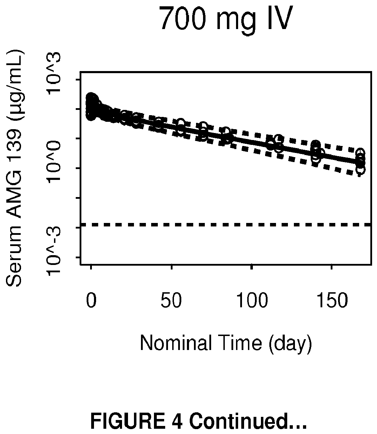

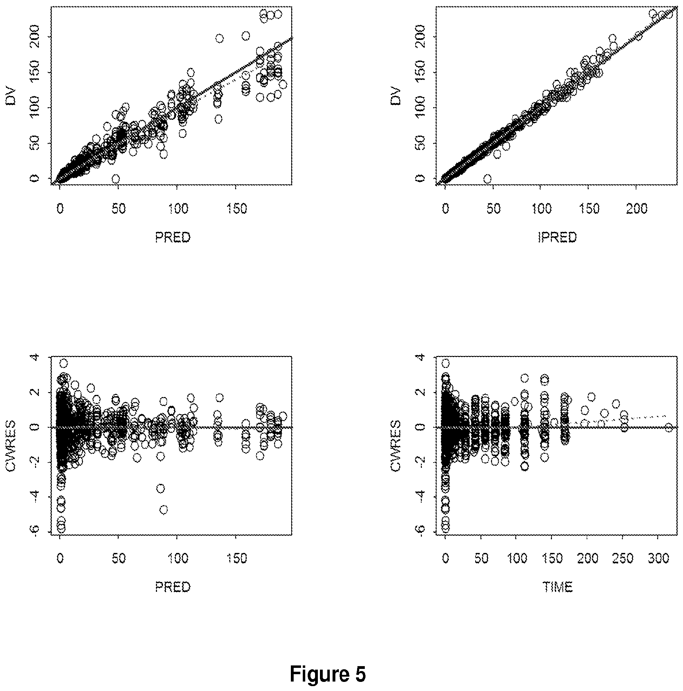

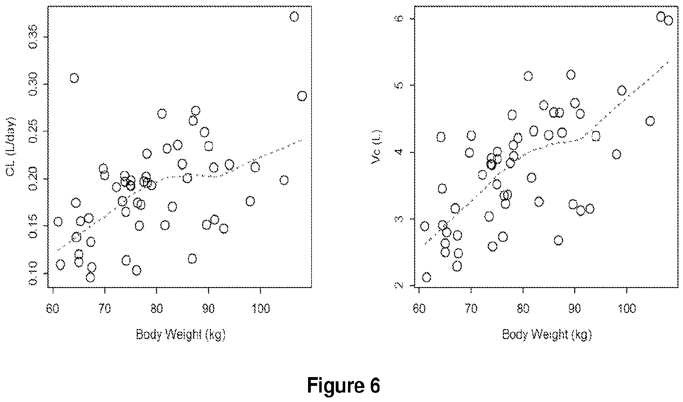

The final AMG 139 pop PK model predicted mean concentration-time profiles that fit the data well within 90% confidence intervals (Figure 4), and visual predictive diagnostic plots show strong correlations between observed and predicted values (Figures 4 and 5). The estimated AMG 139 absorption rate constant, systemic clearance (CL), and central volume of distribution (V_{c}) were 0.242 h⁻¹, 0.171 L/day, and 3.58 L, respectively, with inter-individual variability of 66%, 24%, and 20%, respectively (Table 3). Body weight as a covariate had power coefficient values of 1.04 and 1.11 for CL and V_{c}, respectively, and showed a positive correlation with CL and V_{c} (Figure 6). After adjusting for body weight, the additional effect of a disease status covariate on CL [1.13-fold increase (0.93-1.3, 95% CI)] did not show a statistically significant improvement on the model in this Phase 1 study dataset.

**Table 3: Population PK Model Parameter Estimates after Single Dose Administration of AMG 139 to Healthy Volunteers and Psoriasis Subjects**

| Parameter | Parameter estimate | SE | Inter-individual variability (%) | SE |
|---|---|---|---|---|
| ka (hr⁻¹) | 0.242 | 0.0354 | 66 | 9 |
| CL (L/day) | 0.171 | 0.0149 | 24 | 3 |
| V_{c} (L) | 3.58 | 0.318 | 20 | 2 |
| Vₚ (L) | 3.16 | 0.322 | 25 | 3 |
| Q (L) | 0.576 | 0.107 | 90 | 15 |

The AMG 139 pop PK model established utility for simulating AMG 139 PK in future inflammatory disease populations (e.g. Psoriasis and Crohn's Disease), as well as incorporation with ongoing efficacy studies for establishment of a PK/pharmacodynamic model.

These results support potentially several dosing regimens for administering AMG 139 to an individual afflicted with an inflammatory bowel disease condition that is associated with IL-23 pathway. An appropriate dosing regimen can be selected from the dosing regimens shown in Table 4 below.

**Table 4: Dosing Regimens**

| | |
|---|---|
| 1. | 210 mg SC or IV every 1 month (0.5 - 3 months); 210 mg includes amounts in the range of 150 - 299 mg |
| 2. | 700 mg SC or IV every 3 months (4 - 8 months); 700 mg includes amounts in the range of 300 - 1100 mg |

In view of the above, it will be appreciated that the invention also encompasses the following items:
1. A method of treating Crohn's disease in a subject in need thereof comprising administering to the subject an anti-IL-23 antibody in an amount and at an interval of:
   a. 15 - 54 mg every 0.5 - 1.5 months;
   b. 55 - 149 mg every 1.5 - 4.5 months;
   c. 150 - 299 mg every 4 - 8 months; or
   d. 300 - 1100 mg every 4 - 12 months.
2. The method of item 1, wherein the amount and interval are:
   a. 15 - 21 mg every 0.5 - 1.0 month;
   b. 55 - 70 mg every 1.5 - 3.0months;
   c. 150 - 260 mg every 4 - 6 months; or
   d. 300 - 700 mg every 4 - 8 months.
3. The method of item 1, wherein the amount and interval are:
   a. 21 mg every month;
   b. 70 mg every 3 months;
   c. 210 mg every 6 months; or
   d. 700 mg every 6 months.
4. The method of item 1, wherein the amount and interval are:
   a. 210 mg every 3 months or
   b. 700 mg every 3 months.
5. The method of item 1, wherein the amount and interval are:
   a. 210 mg every 1 month or
   b. 700 mg every 1 month.
6. A method of treating Crohn's disease in a subject in need thereof comprising administering to the subject an amount of an anti-IL-23 antibody in an amount and at an interval sufficient to achieve and/or maintain a quantity of anti-IL-23 antibody per volume of serum of between 12.5 ng/ml and 1000 ng/ml.
7. The method of item 6, wherein the quantity of an anti-IL-23 antibody per volume of serum is at least 10 ng/ml.
8. The method of item 6, wherein the quantity of an anti-IL-23 antibody per volume of serum is selected from the group consisting of: at least 25 ng/ml; at least 50 ng/ml; at least 60 ng/ml; at least 70 ng/ml; at least 75 ng/ml; and at least 80 ng/ml.
9. The method of item 6, wherein the quantity of an anti-IL-23 antibody per volume of serum is between 85 ng/ml and 100 ng/ml.
10. The method of item 6, wherein the quantity of an anti-IL-23 antibody per volume of serum is between 70 ng/ml and 150 ng/ml.
11. The method of item 6, wherein the quantity of an anti-IL-23 antibody per volume of serum is is between 50 ng/ml and 250 ng/ml.
12. The method of item 6, wherein the quantity of an anti-IL-23 antibody per volume of serum is is between 40 ng/ml and 500 ng/ml.
13. The method of item 6, wherein the quantity of an anti-IL-23 antibody per volume of serum is between 25 ng/ml and 750 ng/ml.
14. The method of item 6, wherein the quantity of an anti-IL-23 antibody per volume of serum is between 10 ng/ml and 1,000 ng/ml.
15. The method according to any of the above items , wherein the anti-IL23 antibody is administered IV.
16. The method according to any of the above items , wherein the anti-IL23 antibody is administered SC.
17 The method according to any of the above items, wherein the anti-IL-23 antibody is AMG 139.

## Claims

1. An anti-IL-23 antibody for use in treating Crohn's disease, wherein the anti-IL-23 antibody is administered in an amount and at an interval of:
a. 15 - 54 mg every 0.5 - 1.5 months;
b. 55 - 149 mg every 1.5 - 4.5 months;
c. 150 - 299 mg every 4 - 8 months; or
d. 300 - 1100 mg every 4 - 12 months.

2. The anti-IL-23 antibody for the use according to claim 1, wherein the amount and interval are:
a. 15 - 21 mg every 0.5 - 1.0 month;
b. 55 - 70 mg every 1.5 - 3.0 months;
c. 150 - 260 mg every 4 - 6 months; or
d. 300 - 700 mg every 4 - 8 months.

3. The anti-IL-23 antibody for the use according to claim 1 or claim 2, wherein the amount and interval are:
a. 21 mg every month;
b. 70 mg every 3 months;
c. 210 mg every 6 months; or
d. 700 mg every 6 months.

4. The anti-IL-23 antibody for the use according to claim 1 or claim 2, wherein the amount and interval are:
a. 210 mg every 3 months;
b. 700 mg every 3 months;
c. 210 mg every 1 month; or
d. 700 mg every 1 month.

5. An anti-IL-23 antibody for use in treating Crohn's disease, wherein the anti-IL-23 antibody is administered in an amount and at an interval sufficient to achieve and/or maintain a quantity of anti-IL-23 antibody per volume of serum of
a. between 12.5 ng /ml and 1000 ng/ml; or
b. at least 10 ng/ml.

6. The anti-IL-23 antibody for the use according to claim 5, wherein the quantity of an anti-IL-23 antibody per volume of serum is selected from the group consisting of: at least 25 ng/ml; at least 50 ng/ml; at least 60 ng/ml; at least 70 ng/ml; at least 75 ng/ml; and at least 80 ng/ml.

7. The anti-IL-23 antibody for the use according to claim 5, wherein the quantity of an anti-IL-23 antibody per volume of serum is between 85 ng/ml and 100 ng/ml.

8. The anti-IL-23 antibody for the use according to claim 5, wherein the quantity of an anti-IL-23 antibody per volume of serum is between 70 ng/ml and 150 ng/ml.

9. The anti-IL-23 antibody for the use according to claim 5, wherein the quantity of an anti-IL-23 antibody per volume of serum is between 50 ng/ml and 250 ng/ml.

10. The anti-IL-23 antibody for the use according to claim 5, wherein the quantity of an anti-IL-23 antibody per volume of serum is between 40 ng/ml and 500 ng/ml.

11. The anti-IL-23 antibody for the use according to claim 5, wherein the quantity of an anti-IL-23 antibody per volume of serum is between 25 ng/ml and 750 ng/ml.

12. The anti-IL-23 antibody for the use according to claim 5b, wherein the quantity of an anti-IL-23 antibody per volume of serum is between 10 ng/ml and 1,000 ng/ml.

13. The anti-IL-23 antibody for the use according to any of the above claims, wherein the anti-IL23 antibody is administered IV.

14. The anti-IL-23 antibody for the use according to any of the above claims, wherein the anti-IL23 antibody is administered SC.

15. The anti-IL-23 antibody for the use according to any of the above claims, wherein the anti-IL-23 antibody comprises the heavy and light chain variable regions of SEQ ID NO:1 and SEQ ID NO:2, respectively.
